(19) Europäisches Patentamt
European Patent Office
Office européen des brevets

(11) Publication number : **0 440 347 B1**

# (12) EUROPEAN PATENT SPECIFICATION

(45) Date of publication of patent specification : **08.09.93 Bulletin 93/36**

(51) Int. Cl.$^5$ : **A61K 31/20**

(21) Application number : **91300272.1**

(22) Date of filing : **15.01.91**

## (54) Essential fatty acid compositions for increasing the fat content of mammalian milk.

(30) Priority : **30.01.90 GB 9002048**

(43) Date of publication of application : **07.08.91 Bulletin 91/32**

(45) Publication of the grant of the patent : **08.09.93 Bulletin 93/36**

(84) Designated Contracting States : **AT BE CH DE DK ES FR GB GR IT LI LU NL SE**

(56) References cited :
**WO-A-88/10112**
**DE-A- 3 808 885**

(56) References cited :
**AMERICAN JOURNAL OF CLINICAL NUTRITION, vol. 32, February 1979; B. HALL, pp. 304-312**
**HIGHLIGHTS AGRIC., vol. 28, no. 1, 1981; M. CRAIG-SCHMIDT et al., p. 15**

(73) Proprietor : **SCOTIA HOLDINGS PLC**
**Efamol House Woodbridge Meadows**
**Guildford Surrey GU1 1BA (GB)**

(72) Inventor : **Horrobin, David Frederick**
**c/o Efamol House, Woodbridge Meadows**
**Guildford, Surrey GU1 1BA (GB)**

(74) Representative : **Miller, Joseph et al**
**J. MILLER & CO. 34 Bedford Row, Holborn**
**London WC1R 4JH (GB)**

EP 0 440 347 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

## BACKGROUND

Breast feeding is the preferred method of feeding human infants, providing both energy and essential nutrients for the developing baby. As far as energy supply is concerned, fat is by far the most important constituent of milk. The fat also contains essential fatty acids (EFAs), vitamin-like essential nutrients which are extremely important in many aspects of human structure and function. EFAs seem to be particularly important for the developing brain, immunological system and cardiovascular system although they have roles to play in every organ in the body.

During prolonged breast feeding, the total fat and the EFA contents of human milk tend to fall progressively. These falls may limit the success of breast feeding and lead to an early requirement for supplementation with other foods. We have now discovered a method of reducing or preventing the fall of breast milk fat levels during prolonged breast feeding and of in many cases actually increasing both the total fat and the EFA content of human milk.

The outline of n-6 series fatty acid conversion in the body is:-

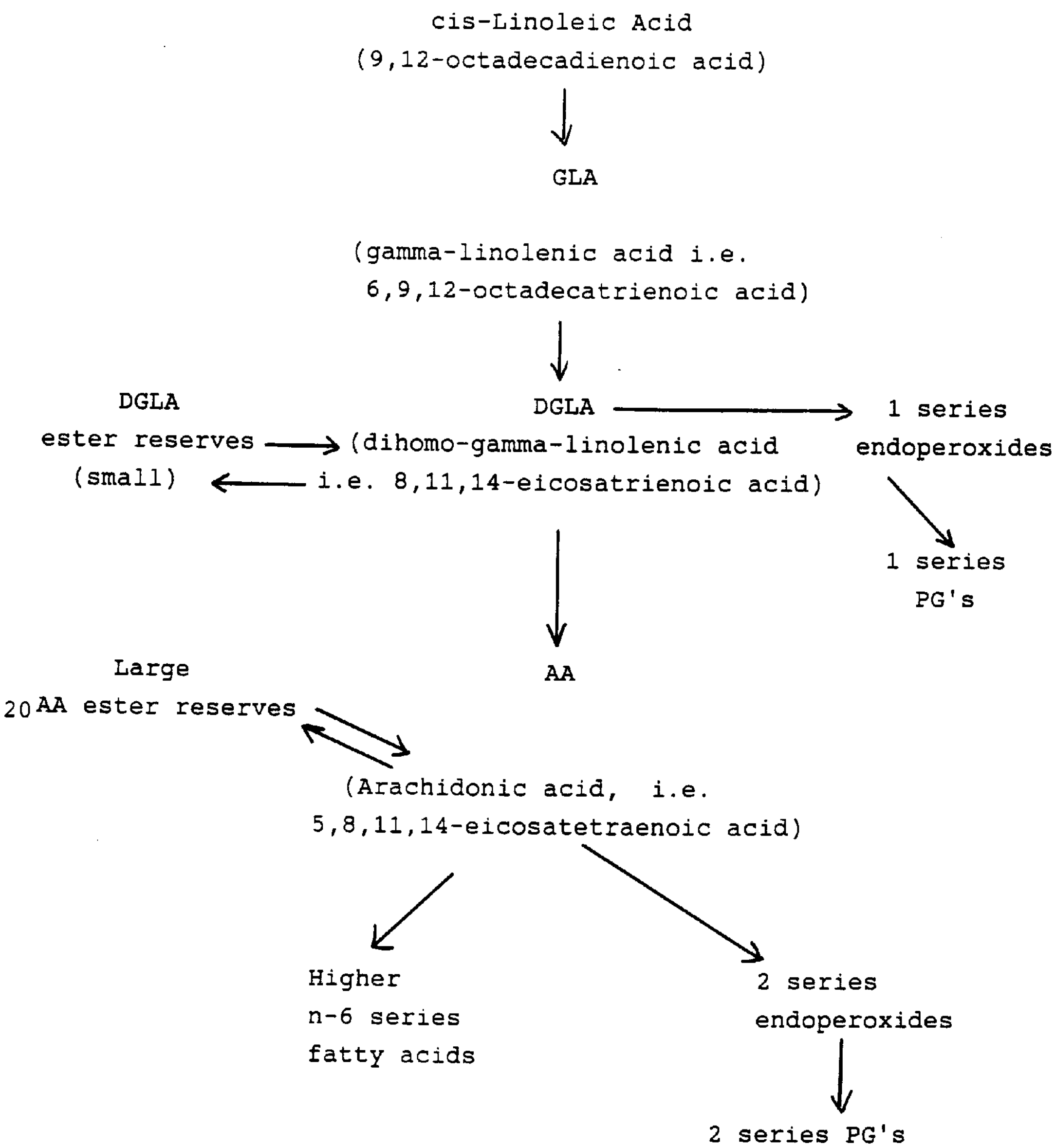


As appears from the chart, linoleic acid (LA) is the main n-6 EFA in the diet but in order to be useful to the body it must be converted first to gamma-linolenic acid (GLA) and then to further metabolites such as dihomo-gamma-linolenic acid (DGLA) and arachidonic acid (AA) which are precursors of 1 and 2 series prostaglandins respectively. GLA is very rapidly converted to DGLA and the two may be regarded as metabolically equivalent. However, the formation of GLA itself is slow and rate-limited by the 6-desaturase enzyme acting to generate it from LA. Similarly the conversion of DGLA to AA is also relatively slow.

## EXPERIMENTAL

The production of breast milk and milk fat is stimulated by the hormone prolactin. Prolactin works in part by stimulating the formation of prostaglandin PGE1 from DGLA, and in view of the frequently slow production

of its precursor GLA from linoleic acid in the body that the action of prolactin on the breast may be amplified by providing GLA or DGLA on which the stimulating mechanism can act. In a preliminary study in three nursing mothers GLA in the form of evening primrose oil appeared to stimulate the production of human milk fat. We therefore set up a placebo-controlled study in thirty six nursing mothers who wished to breast feed for a prolonged period: they had already completed three to four months feeding and aimed to continue for another eight months. Of this group, eighteen were given each day for eight months 8 x 500 mg capsules of evening primrose oil containing a total of 320 mg of GLA. The other eighteen were correspondingly given identical-appearing placebo capsules containing liquid paraffin.

At the beginning and end of the period a morning milk sample was collected from each mother. The total lipid was extracted and its amount measured. The fatty acids in the lipid were then converted to their methyl esters and analysed by gas chromatography. The results are shown in the accompanying table, in g/l ± SD:-

TABLE 1

| Fatty Acid | Baseline | End | Change |
|---|---|---|---|
| | **On evening primrose oil** | | |
| Total fat | 44.26 ± 19.84 | 50.96 ± 28.37 | + 15.1% |
| Linoleic | 5.86 ± 2.47 | 8.19 ± 5.12 | + 39.8% |
| GLA + DGLA | 0.10 ± 0.05 | 0.22 ± 0.12 | + 120% |
| Arachidonic | 0.26 ± 0.13 | 0.28 ± 0.16 | + 7.7% |
| | **On placebo** | | |
| Total fat | 41.21 ± 11.75 | 31.83 ± 19.43 | - 22.8% |
| Linoleic | 7.19 ± 2.94 | 5.21 ± 2.79 | - 27.5% |
| GLA + DGLA | 0.18 ± 0.07 | 0.07 ± 0.04 | - 61.1% |
| Arachidonic | 0.23 ± 0.12 | 0.17 ± 0.16 | - 26.1% |

As can be seen, in the placebo group there was a decline both in the total fat level and in the levels of the individual EFAs in the milk. In contrast, in the GLA-treated group, not only was the decline prevented it was actually reversed. The GLA supplement increased both the total fat and the EFA content of the milk and especially the beneficial GLA/DGLA content. This effect in quality is significant, but since fat is the major source of energy in milk, the GLA supplement also substantially increased the total energy content of the milk.

The effect is not one of mere appearance of-the supplement or its metabolites DGLA and AA in the milk, but a real effect on fat metabolism. This is shown by the linoleic acid content, given that in the body the conversions of linoleic acid to GLA, GLA to DGLA, DGLA to AA, and so on are not reversible.

Further, although not measured formally, we have had reports from several mothers that the milk flow increased substantially while taking evening primrose oil. GLA therefore stimulates milk flow as well as increasing EFA and total fat content.

**STATEMENT OF INVENTION**

The above discussion is in terms of human milk production but the physiology of milk production is similar in mammals generally and the invention is therefore applicable in veterinary practice where improvement of the fat quantity and composition and of the flow of milk is desired, and particularly in animal husbandry where for example cattle, sheep, goats are kept for commercial milk production.

The invention therefore lies in a method of preparation of a medicament for increasing one or more of the total fat and therefore energy content of mammalian and particularly human milk

and the flow of that milk, or for preventing or reducing the normal fall in milk fat content that occurs during prolonged lactation, wherein GLA and/or DGLA is provided, alone or in a physiologically acceptable diluent or carrier, in a form administrable to the lactating female.

Conveniently the medicament is suited to daily administration of 1 mg to 10 g of GLA and/or DGLA (such amount being the total if both are present) per 70 kg body weight, preferably 50 mg to 3 g, and very preferably 200 mg to 1.5 g, the medicament being in dosage unit form containing said amounts or sub-multiples thereof for oral, parenteral or other internal administration or as a topical preparation containing 0.01 to 20 weight % GLA and/or DGLA (said amount being the total if both are present) for application preferably to the mammae.

The invention also provides a method of increasing one or more of the total fat and therefore energy content of mammalian and particularly human milk

and the flow of that milk, or for preventing or reducing the normal fall in milk fat content that occurs during prolonged lactation, wherein GLA and/or DGLA is administered to the lactating female, conveniently in the daily amounts and in the forms set out above.

**DERIVATIVES OF EFAs**

The acids may be used as such or as pharmaceutically acceptable and physiologically equivalent derivatives as for example detailed later herein and reference to any of the acids including reference in the claims is to be taken as including reference to the acids when in the form of such derivatives. Equivalence is demonstrated by entry into the biosynthetic pathways of the body as evidenced by effects corresponding to those of the acids themselves or their natural glyceride esters. Thus, indirect identification of useful derivatives is by their having the valuable effect in the body of the acid itself, but conversion for example of GLA to DGLA and on to AA can be shown directly by gas chromatographic analysis of concentrations in blood, body fat, or other tissue by standard techniques, for example those of Pelick et al, page 23, "Analysis of Lipids and Lipoproteins" Ed Perkins, American Oil Chemists Society, Champaign, Illinois, U.S.A.

In outline the method is suitably that plasma samples (1 ml) are extracted with chloroform:methanol (2:1). The extract is filtered through sodium sulphate, evaporated to dryness, and taken up in 0.5 ml chloroform:methanol. The lipid fractions are separated by thin layer chromatography or silica gel plates. The phospholipid fraction, taken to reflect essential fatty acid contents most sensitively, is methylated using boron trifluoride-methanol. The resulting methyl esters of the fatty acids are separated and measured using a Hewlett-Packard 5880 gas chromatograph with a six foot column packed with 10% Silar (RTM) on Chromosorb (RTM) WAW 106/230. The carrier gas is helium (30 ml/min). Oven temperature is programmed to rise from 165°C to 190°C at 2°C/min. Detector temperature is 220°C and injector temperature 200°C. Retention times and peak areas are automatically computed by Hewlett-Packard Level 4 integrator. Peaks are identified by comparison with standard fatty acid methyl esters.

**DIETARY COMPOSITIONS**

The invention is chiefly described in terms of medicaments and methods of treatment but it will be understood that the gamma-linolenic and other EFAs, being in the nature of dietary supplements, can be incorporated in a dietary margarine or other foodstuff or animal feed and such are to be understood as within the term pharmaceutical composition or medicament when used herein (including the claims).

**FORMS AND SOURCES OF GAMMA-LINOLENIC AND OTHER ACIDS**

Convenient physiologically equivalent derivatives of GLA and DGLA for use according to the invention include salts, amides, esters including glyceride esters and alkyl (e.g. $C_1$ to $C_4$) esters, and phospholipids.

If desired, pharmaceutical compositions may be produced for use in the invention by associating the natural or synthetic acids, as such or as derivatives, with an acceptable pharmaceutical vehicle. It is, however, at present convenient to provide at least GLA in the form of an available oil having a high GLA content, hence reference to "oils" herein.

One source of oils currently available is the seed of evening primrose species such as Oenothera biennis L. and Oenothera lamarckiana, the oil extract therefrom containing about 8% GLA and about 72% linoleic acid in the form of their glycerides, together with other glycerides (percentages based on total fatty acids). Other sources of GLA are borage species such as Borago officinalis which, though current yield per acre is low, provide a richer source than Oenothera oil. Oils from the seeds of members of the Ribes family are also often

rich in GLA. Recent studies on fungi which can be cultivated by fermentation promise a fungal oil source, and chemical synthesis is also possible.

The oil is extracted from the seed by one of the conventional methods of extraction such as cold pressure, screw pressure after partially cooking the seed, or solvent extraction.

Fractionation of a typical sample of this oil in the form of methyl esters shows the relative proportions:

| | |
|---|---|
| Palmitate | 6.15 |
| Stearate | 1.6 |
| Oleate | 10.15 |
| Linoleate | 72.6 |
| Gamma-linolenate | 8.9 |

The seed oil extracts referred to above can be used as such or can, for example, if desired, be fractionated to yield an oily composition containing the triglycerides of gamma-linolenic and linoleic acids as the main fatty acid components, the gamma-linolenic acid content being, if desired, a major proportion. Seed oil extracts appear to have a stabilising effect upon DGLA if present.

If DGLA is to be used it can be prepared by chemical synthesis or by fungal fermentation.

## PHARMACEUTICAL PRESENTATION

As mentioned briefly above, the compositions are conveniently in a form suitable for oral, rectal, parenteral or other internal administration in a suitable pharmaceutical vehicle, as discussed in detail, for example, in Williams British Patent Specification No. 1,082,624, to which reference may be made, and in any case very well known generally for any particular kind of preparation. Thus, for example, tablets, capsules, ingestible liquid or powder preparations can be prepared as required, and topical preparations also when the gamma-linolenic acid or other acids are absorbed through the skin. Injectable solutions of hydrolysed Oenothera oil may be prepared using albumin to solubilise the free acid.

Advantageously, a preservative is incorporated into the preparation. Alpha-tocopherol in concentration of about 0.1% by weight has been found suitable for the purpose and is one of a number of possible stabilisers well known in the field and including also for example ascorbyl palmitate and stearate.

It will be understood that the absolute quantity of active materials present in any dosage unit should not exceed that appropriate to the rate and manner of administration to be employed but on the other hand should also desirably be adequate to allow the desired rate of administration to be achieved by a small number of doses. The rate of administration will moreover depend on the precise pharmacological action desired.

## EXAMPLES

The following may be given to nursing mothers for increasing the total fat and therefore energy content of breast milk:-

1. 8 x 500 mg capsules per day evening primrose oil (containing 8% GLA by weight).
2. 6 x 1 g capsules per day borage oil (containing 22% GLA by weight).
3. 8 x 500 mg capsules per day blackcurrant seed oil (containing 18% GLA by weight).
4. 6 x 500 mg capsules per day oil of microbial origin from the fungi Mortierella or Rhizopus containing 20% GLA by weight. (The biomass of suitable strains contains ca. 15% oil by weight of which 15 to 20% is GLA).
5. 6 x 500 mg capsules per day oil of such microbial origin containing 19% of DGLA by weight.
6. 2 x 250 mg capsules per day purified GLA in triglyceride, free fatty acid, methyl ester or ethyl ester form.
7. 2 x 300 mg capsules per day DGLA in triglyceride, free fatty acid, methyl or ethyl ester form.
8. Cream for application to the breasts containing 20% evening primrose oil by weight.
9. Cream for application to the breasts containing 2% of purified GLA or DGLA by weight.
10. In animal husbandry corresponding amounts of GLA or DGLA related to body weight may be given, for example GLA may be used in the form of evening primrose seed cake residue (3% residual oil of which 9% is GLA), or as crushed evening primrose, borage or blackcurrant seeds, or as microbial biomass, for example:-
    a. A cake for feeding to cattle containing 2% by weight of evening primrose seed cake.
    b. A cake for feeding to cattle containing 1% by weight of crushed borage seed Borago officinalis (24% oil, of which 21% is GLA).
    c. A cake for feeding to goats containing 0.5% by weight of GLA-rich microbial biomass, Mortierella or Rhizopus as above.

## Claims

1. Use of a composition comprising GLA and/or DGLA or physiologically equivalent derivatives thereof, alone or in a physiologically acceptable diluent or carrier, in a form administrable to the lactating female for increasing one or more of the total fat and therefore energy content of mammalian and particularly human milk, and the flow of that milk, or for preventing or reducing the normal fall in milk fat content that occurs during prolonged lactation.

2. The use according to claim 1 wherein the composition is suited to daily administration of 1 mg to 10 g of GLA and/or DGLA or physiologically equivalent derivatives thereof (such amount being the total if both are present) per 70 kg body weight, preferably 50 mg to 3 g, and very preferably 200 mg to 1.5 g, for oral, parenteral or other internal administration or as a topical preparation containing 0.01 to 20 weight % GLA and/or DGLA (said amount being the total if both are present) for application preferably to the mammae.

3. A method of increasing one or more of the total fat and therefore energy content of mammalian and particularly human milk, and the flow of that milk, or for preventing or reducing the normal fall in milk fat content that occurs during prolonged lactation, wherein GLA and/or DGLA or physiologically equivalent derivatives thereof is administered to the lactating female.

4. The method of claim 3 wherein the daily amounts set out in claim 2 are administered as set out in that claim.

## Patentansprüche

1. Verwendung eines Präparate, das GLA und/oder OGLA oder physiologisch äquivalente Derivate enthält, allein oder in einem physiologisch akzeptierbaren Verdünnungsmittel oder Träger in einer Form, die stillenden Frauen verabreicht werden kann, zur Erhöhung eines oder mehrerer der gesamten Fette und daher des Energiegehalts von Säugetier- und besonders menschlicher Milch und des Flusses dieser Milch, oder zur Verhinderung oder zur Reduzierung des normalen Rückgangs des Milchfettgehaltes, der während längeren Stillers auftritt.

2. Verwendung gemäß Anspruch 1, wobei das Präparat für tägliche Verabreichung von 1 mg bis 10 g GLA und/oder DGLA oder physiologisch äquivalenten Derivaten davon (wobei solche Mengen die Summe von beiden sind, falls vorhanden) pro 70 kg Körpergewicht, vorzugsweise 50 mg bis 3 g, und noch besser 200 mg bis 1,5 g für orale, parentorale oder sonstige, interne Verabreichung geeignet ist oder als Oberflächenpräparat, das 0,01 bis 20 Gew. % GLA und/oder DGLA (wobei die genannte Menge die Summe ist, wenn beide vorhanden sind) enthält, zur Anwendung vorzugsweise an den Mammae.

3. Eine Methode zur Erhöhung eines oder mehrerer der gesamten Fette und daher des Energiegehaltes von Säugetier- und besonders menschlicher Milch und des Flusses dieser Milch oder zur Verhinderung oder Reduzierung des normalen Rückgangs des Milchfettgehaltes, der während längeren Stillens auftritt, wobei GLA und/oder DGLA oder physiologisch äquivalente Derivate davon der stillenden Frau verabreicht werden.

4. Eine Methode gemäß Anspruch 3, wobei die täglichen Mengen, die in Anspruch 2 dargelegt sind, verabreicht werden, wie in diesem Anspruch dargelegt.

## Revendications

1. Utilisation d'une composition comprenant de l'acide γ-linolénique (GLA) et/ou de l'acide dihomo-γ-linolénique (DGLA) ou un de leurs dérivés physiologiquement équivalents, seuls ou dans un diluant ou support physiologiquement acceptables, sous une forme administrable à une femelle allaitante pour augmenter la teneur en graisse totale et par conséquent en énergie du lait des mammifères, et en particulier du lait humain, et/ou le débit de ce lait, ou pour empêcher et/ou pour réduire, la baisse normale de la teneur en graisse du lait qui se produit au cours d'une lactation prolongée.

2. Utilisation selon la revendication 1, dans laquelle la composition convient pour l'administration quotidienne

de 1 mg à 10 g de GLA et/ou de DGLA ou d'un de leurs dérivés physiologiquement équivalents (cette quantité étant la somme des deux s'ils sont tous deux présents) pour un poids corporel de 70 kg, de préférence de 50 mg à 3 g, et plus préférablement de 200 mg à 1,5 g pour l'administration orale, parentérale, ou une autre administration interne, ou sous forme de préparation locale contenant 0,01 à 20 % en poids de GLA et/ou DGLA (cette quantité étant la somme des deux s'ils sont tous deux présents), pour l'administration de préférence à des mammifères.

**3.** Procédé pour augmenter la teneur en graisse totale et par conséquent en énergie du lait des mammifères, et en particulier du lait humain, et le débit de ce lait, et/ou pour empêcher ou réduire la baisse normale de la teneur en graisse du lait qui se produit au cours d'une lactation prolongée, dans lequel le GLA et/ou DGLA ou leurs dérivés physiologiquement équivalents sont administrés à la femelle allaitante.

**4.** Procédé selon la revendication 3, dans lequel les quantités quotidiennes indiquées dans la revendication 2 sont administrées comme il est indiqué dans cette revendication.